# EUROPEAN PATENT SPECIFICATION

(11) **EP 4 565 210 B1**
(45) Date of publication and mention of the grant of the patent: **02.09.2026**
(21) Application number: 24701341.0
(22) Date of filing: 17.01.2024
(51) Int. Cl.: A61K 9/127, A61K 31/7032, A61P 25/16

(54) **LIPOSOMAL COMPOSITION FOR USE IN A METHOD OF TREATING PARKINSON'S DISEASE**
LIPOSOMALE ZUSAMMENSETZUNG ZUR VERWENDUNG IN EINEM VERFAHREN ZUR BEHANDLUNG VON MORBUS PARKINSON
COMPOSITION LIPOSOMALE POUR UTILISATION DANS UNE MÉTHODE DE TRAITEMENT DE LA MALADIE DE PARKINSON

(30) Priority: 18.01.2023 EP 23152217
(43) Date of publication of application: 11.06.2025
(73) Proprietor: InnoMedica Holding AG, 6300 Zug (CH)
(72) Inventor: HALBHERR, Stéfan Jonathan, 3084 Wabern (CH); PEITSCH, Camille Françoise, 3178 Bösingen (CH)
(74) Representative: Hepp Wenger Ryffel AG
(86) International application number: PCT/EP2024/051071
(87) International publication number: WO 2024/153712

(56) References cited:
- WO-A1-2019/122220
- SCHNEIDER J S: "Parkinson's disease: Improved function with GM1 ganglioside treatment in a randomized placebo-controlled study", NEUROLOGY, vol. 50, no. 6, 1 June 1998 (1998-06-01), pages 1630 - 1636, XP093290948
- SCHNEIDER JAY S. ET AL: "GM1 ganglioside in Parkinson's disease: Results of a five year open study", JOURNAL OF NEUROLOGICAL SCIENCES., vol. 292, no. 1-2, 5 March 2010 (2010-03-05), NL, pages 45 - 51, XP093007787, ISSN: 0022-510X, DOI: 10.1016/j.jns.2010.02.009
- SCHNEIDER JAY S. ET AL: "GM1 ganglioside modifies microglial and neuroinflammatory responses to [alpha]-synuclein in the rat AAV-A53T [alpha]-synuclein model of Parkinson's disease", MOLECULAR AND CELLULAR NEUROSCIENCES., vol. 120, May 2022 (2022-05-01), US, pages 103729, XP093057208, ISSN: 1044-7431, DOI: 10.1016/j.mcn.2022.103729

## Description

The present invention relates to a liposomal composition for use in a method of treating Parkinson's disease.

Parkinson's disease (PD) is a brain disorder that causes unintended or uncontrollable movements, such as shaking, stiffness, and difficulty with balance and coordination. Symptoms usually begin gradually and worsen over time. As the disease progresses, people may have difficulty walking and talking. They may also have mental and behavioural changes, sleep problems, depression, memory difficulties, and fatigue.

The most prominent signs and symptoms of PD occur when neurons in the basal ganglia, an area of the brain that controls movement, become impaired and/or die. Normally, these neurons produce an important brain chemical known as dopamine. When the neurons die or become impaired, they produce less dopamine, which causes the movement problems associated with the disease.

Although there is no cure for PD, medicines, surgical treatment, and other therapies can often relieve some symptoms. Medicines can help treat the symptoms of Parkinson's by increasing the level of dopamine in the brain, by having an effect on other brain chemicals, such as neurotransmitters, which transfer information between brain cells, or by helping control non-movement symptoms.

The main therapy for Parkinson's is levodopa (or L-DOPA). Nerve cells use levodopa to make dopamine to replenish the brain's dwindling supply. Usually, people take levodopa along with another medication called carbidopa. Carbidopa prevents or reduces some of the side effects of levodopa therapy - such as nausea, vomiting, low blood pressure, and restlessness - and reduces the amount of levodopa needed to improve symptoms.

Other medicines to treat Parkinson's symptoms include dopamine agonists to stimulate the production of dopamine in the brain, enzyme inhibitors **(e.g.,** MAO-B inhibitors, COMT inhibitors) to increase the amount of dopamine by slowing down the enzymes that break down dopamine in the brain, amantadine to help reduce involuntary movements and anticholinergic drugs to reduce tremors and muscle rigidity.

Certain types of glycolipids have been identified to play a key role in the development and treatment of PD. Glycolipids are lipids with a carbohydrate attached by a glycosidic bond. Their role in living cells is to maintain the stability of the cell membrane and to facilitate cellular recognition. Glycosphin-golipids are a subtype of glycolipids containing the amino alcohol sphingosine. They occur in the nervous system and elsewhere in the body and they are involved in metabolic and pathological changes that accompany PD.

Ganglioside GM1 (monosialotetrahexosylganglioside, CAS: 37758-47-7) is a glycosphingolipid consisting of a branched pentasaccharide made up from one sialyl residue, two galactose residues, one *N*-acetylgalactosamine residue and a glucose residue at the reducing end attached to *N*-stearoylsphingosine via a beta-linkage. GM1 has important physiological properties and impacts neuronal plasticity and repair mechanisms, and the release of neu-rotrophins in the brain (VASQUES, J.F. et al. Gangliosides in nervous system development, regeneration, and pathologies. Neural Regen. Res. 2023, Vol. 18, No. 1, pages 81 to 86). It is an amphiphilic, endogenous molecule found abundantly on the outer leaflets of membranes of all mammalian cells and is particularly enriched on the neuronal plasma membrane (it constitutes 5 % ± 10 % of the total lipid mass).

GM1 interacts with the neighboring proteins to modulate intracellular signaling, mainly of biochemical pathways of neuronal differentiation, homeostasis, protection, and restoration. It has been reported to have neurotrophic properties and influences a variety of cellular activities at the level of the plasma membrane as well as intracellularly. In the central nervous system (CNS), GM1 exhibits many ganglioside functions during development, function and repair. It may also counteract neuroinflamma-tion.

Higher gangliosides like GM1 appear only during late stages of organogenesis and foetal growth, hinting towards their integral function to guide formation of a fully functional and young CNS. The possibility of reintroducing this "young CNS"-state - at least in part - from a lipid-based perspective could have therapeutic applications (XU, Y.-H. et al. Multi-system disorders of glycosphingolipid and ganglioside metabolism. J. Lipid Res. 2010, Vol. 51, No. 7, pages 1643 to 1675).

GM1 belongs to the a-series gangliosides and its biosynthesis occurs in the Golgi apparatus. Along with the more complex gangliosides, GM1 is enriched in the pre- and postsynaptic membranes of the synaptic terminals. In addition to regulating nuclear Ca²⁺-homeostasis (via high-affinity binding of the ganglioside to the sodium/calcium exchanger at the nuclear envelope) and other cellular functions, GM1 modulates neurotrophic activity by associating with receptors TrkA, TrkB, and GDNF (NEWBURN, E.N. et al. GM1 ganglioside enhances Ret signaling in striatum. J. Neurochem. 2014, Vol. 130, No. 4, pages 541 to 554).

The role of GM1 in both the generation and potential treatment of PD has been of particular interest (CHOWDHURY, S. et al. The Key Role of GM1 Ganglioside in Parkinson's Disease. Biomolecules 2022, Vol. 12, No. 173, pages 1 to 9). GM1 deficiency prevents the normal functioning of cells dependent on an adequate level of this ganglioside, resulting in their gradual degeneration and eventual death. Studies in humans suggest GM1 deficiency could be a contributing factor to the development of idiopathic PD. Reductions in GM1 have been reported in the PD post-mortem brain (WU, G. det al. Deficiency of ganglioside GM1 correlates with Parkinson's disease in mice and humans. J. Neurosci. Res. 2012, Vol. 90, No. 10, pages 1997 to 2008). It was further reported that GM1 levels decrease over time in the healthy human brain. In the brain of a PD patient, however, this decrease is particularly pronounced (HUEBECKER, M. et al. Reduced sphingolipid hy-drolase activities, substrate accumulation and ganglioside decline in Parkinson's disease. Mol. Neurodegener. 2019, Vol. 14, No. 1, pages 1 to 21). It has further been shown that inhibition of microglial activation and potentially damaging neuroinflamma-tion by GM1 ganglioside administration may be among the many factors that contribute to the neuroprotective effects of GM1 in human PD (SCHNEIDER, J. S. et al. GM1 ganglioside modifies microglial and neuroinflammatory responses to α-synuclein in the rat AAV-A53T α-synuclein model of Parkinson's disease. Mol. Cell Neurosci. 2022, Vol. 120, page 103729).

A decrease in GM1 levels has also been observed in ante-mortem cerebrospinal fluid (CSF) of PD patients (compared to age-matched controls), although it was not significant (17.4 % reduction). Similar analysis in blood serum did find a significant reduction in samples from PD patients (LEDEEN, R. et al. Systemic deficiency of GM1 ganglioside in Parkinson's disease tissues and its relation to the disease etiology. Glycoconj. J. 2022, Vol. 39, No. 1, pages 75 to 82).

Transgenic mice wholly or partially deficient in the GM1 family of gangliosides due to disruption of the B4galnt1 gene (GM2 syn-thase-B4galnt1) have been reported to develop PD features based on behavioural and neuropathological criteria. These include movement deficits (as early as five weeks of age) and histopathological details such as depletion of striatal dopamine, loss of dopamine neurons of the substantia nigra pars compacta, and accumulation of alpha synuclein. Notably, the administration of GM1 ameliorated these pathological CNS manifestations (WU, G. et al. Mice deficient in GM1 manifest both motor and non-motor symptoms of Parkinson's disease; successful treatment with synthetic GM1 ganglioside. Exp. Neurol. 2020, Vol. 329, 113284).

There have been a number of clinical investigations exploring the therapeutic potential of GM1 in PD cohorts. The first was a small pilot study. This was an open-label study in which 10 PD patients received 1000 mg of GM1 by intravenous infusion after their last of three baseline functional assessments, and then self-administered GM1 at a dose of 200 mg/day, by subcutaneous injection, for 18 weeks. Under these conditions, GM1 ganglioside proved to be safe and well tolerated. There were no serious adverse events and none of the patients developed elevated anti-GM1 antibody titres (SCHNEIDER, J.S. et al. GM1 ganglioside treatment of Parkinson's disease: an open pilot study of safety and efficacy. Neurology 1995, Vol. 45, No. 6, pages 1149 to 1154).

That study was followed up by a larger randomized placebo-controlled study involving 45 individuals with mild to moderate PD (using the same GM1 treatment regime as above). In this study, significant differences between groups in the Unified PD Rating Scale (UPDRS) motor scores (p=0.0001) and activities of daily living portion (off-period assessment; p=0.04) were reported at 16 weeks (SCHNEIDER, J.S. et al. Parkinson's disease: improved function with GM1 ganglioside treatment in a randomized placebo-controlled study. Neurology 1998, Vol. 50, No. 6, pages 1630 to 1636). In a follow-up open-extension study, at the end of 5 years of GM1 use, the investigators concluded that "long-term GM1 use by PD patients is safe and may provide some clinical benefit". Participants generally had lower UPDRS motor scores (assessed during a practically defined "off" period) (SCHNEIDER, J.S. et al. GM1 ganglioside in Parkinson's disease: Results of a five-year open study. J. Neurol. Sci. 2010, Vol. 292, No. 1, pages 45 to 51).

These findings supported the initiation of a larger, delayed start study. Of the 157 individuals screened, 77 were randomized to either early-start or delayed-start of GM1 treatment. The GM1 treatment regime was the same as the previous studies. A separate comparison group of 17 individuals were used as a standard care treatment comparator. At week 24, the early-start group had significant improvement in UPDRS motor scores vs. a significant worsening of scores in the delayed-start group. The early-start group also showed a sustained benefit vs. the delayed-start group at week 72 and at week 120. Both groups exhibited significant symptom worsening during the washout period after 2 years of GM1 treatment. The study demonstrated that long-term administration of GM1 was safe and well tolerated and it reported no increase in anti-GM1 antibody titres in the participants (SCHNEIDER, J.S. et al. A Randomized, Controlled, Delayed Start Trial of GM1 Ganglioside in Treated Parkinson's Disease Patients. J. Neurol. Sci. 2013, Vol. 324, No. 1, pages 140 to 148). 15 participants from the early-start group and 14 participants from the delayed-start group volunteered to be part of a PET (11)C-methylphenidate imaging study (along with the 11 members of the comparator group). The three groups were scanned at baseline, week 24, and 1-2 years later. The results indicated significant slowing of binding loss in several striatal regions of the GM1-treated groups. In some cases, an increased binding was detected after GM1 use (SCHNEIDER, J.S. et al. GM1 ganglioside in Parkinson's disease: Pilot study of effects on dopamine transporter binding. J. Neurol. Sci. 2015, Vol. 356, No. 1, pages 118 to 123).

One of the problems with translating the therapeutic potential of GM1 from preclinical models to the clinic is the ability of this molecule to adequately reach the CNS when administered peripherally. CNS penetrance is severely limited by GM1's am-phiphilicity, which hampers the passage across the blood-brain barrier. Thus, novel methods of delivery of GM1 to the CNS are required for translation to the clinic.

Furthermore, despite the promising signs of the studies on GM1, certain side effects (adverse events) stand in the way of GM1's marketability, which need to be overcome. For example, pain, irritation of the skin site and severe haematoma at the injection site occur during treatment with GM1, as the most common mode of administration of GM1 is subcutaneous.

For better bioavailability of GM1 in the CNS, the use of liposomes as vesicles for drug delivery for treating PD has been proposed (WO 2019122220 A1 (INNOMEDICA HOLDING AG) 27.06.2019, pages 7 to 8). However, therapeutically effective formulations and dosing regimens do not yet exist in the state of the art.

It is thus an object of the present invention to address those needs and to provide an improved treatment of PD. In particular, it is an object to reduce the adverse events profile frequently encountered with the frequent subcutaneous administration.

The problems have been solved by using a liposomal GM1 formulation as a medicament, having the features according to the independent claim.

The invention relates to a liposomal composition for use in a method of treating PD, said liposomal composition comprising sphingomyelin in a lipid bilayer and a therapeutically effective amount of monosialotetrahexosylganglioside (GM1), wherein a therapeutically effective dose of said liposomal composition is administered at most every 4 days in a primary mode of administration with at least 3 days between each administration; preferably at most every 6 days in a primary mode of administration with at least 5 days between each administration; most preferably at most every 7 days in a primary mode of administration with at least 6 days between each administration.

The monosialotetrahexosylganglioside (GM1) is partially located in a membrane and partially located in an aqueous core of liposomes.

A liposomal composition is a composition containing liposomes in addition to other components, wherein one or more of said other components are encapsulated in the liposomes. A liposome is a spherical vesicle having at least one lipid bilayer. Liposomes may also be multivesicular liposomes in which one vesicle contains one or more smaller vesicles. The liposome has an aqueous solution core surrounded by a hydrophobic membrane in the form of a lipid bilayer. Liposomes have the potential to provide controlled release of an encapsulated active pharmaceutical ingredient (API) over an extended period, and to reduce the side effects of the encapsulated ingredients, by limiting the concentration of free ingredients in the blood stream and tissue. Liposomes can also alter the tissue distribution and uptake of APIs, in a therapeutically favorable way, and can increase the convenience of therapy, by allowing less frequent drug administration. For example, liposomes may transport encapsulated APIs directly to the disease site. The active component can be directly released from the liposome at the treatment site. Thus, a lower dosage of the active component is required, and side effects are in consequence limited.

In the case of PD, liposomes comprising an appropriate lipid membrane composition enhance penetration of the blood-brain barrier. The blood-brain barrier comprises the blood vessels that vascularize the CNS and that tightly regulate the movement of ions, molecules, and cells between the blood and the CNS. This leads to increased deposition of APIs that are encapsulated in liposomes in the CNS.

The preparation of the liposomal composition containing GM1 was described in the prior art (WO 2019122220 A1 (INNOMEDICA HOLDING AG) 27.06.2019). In particular, the circularity of the liposomes used for the liposomal composition of the present invention is greater than or equal to 0.95, in particular 0.98-1.00. The circularity of the liposomes in a formulation are determined by cryogenic transmission electron microscopy (CryoTEM).

The lipids from the lipid membrane composition used to form the liposomes of the present invention are all omnipresent in healthy brain tissue. The liposomes of the present invention comprise the lipid sphingomyelin, which belongs to the groups of phospholipids and sphingolipids. It makes up about 10 % of the lipids of the brain. Sphingomyelin tends to be in greatest concentrations in the plasma membrane, and especially in the outer leaflet, of biological cells.

As it is understood in the art, a lipid bilayer in a liposome is a thin polar membrane made of two layers of lipid molecules, wherein, in aqueous media, the polar, hydrophilic ends of the lipid molecules are on the top and bottom surface of the membrane and the nonpolar, hydrophobic ends of the lipid molecules are inside the membrane. This membrane forms the shell of a sphere with an aqueous core. An encapsulated compound in a liposome may either be located in the aqueous core of the liposome or it may be located in or at the surface of the membrane.

The API of the present invention, GM1, is partially located in the membrane and partially in the aqueous core of the liposome. As described earlier, it is known in the art that ganglioside GM1 plays a big role in both the generation and potential treatment of PD. The chemical name of GM1 according to the International Union of Pure and Applied Chemistry (IUPAC) is (2*S*,4*S*,5*R*,6*R*)-5-acetamido-2-[(2*S*,3*R*,4*R*,5*S*,6*R*)-5-[(2*S*,3*R*,4*R*,5*R*,6*R*)-3-acetamido-5-hydroxy-6-(hydroxymethyl)-4-[(2*R*,3*R*,4*S*,5*R*,6*R*)-3,4,5-trihydroxy-6-(hydroxymethyl)oxan-2-yl]oxyoxan-2-yl]oxy-2-[(2*R*,3*S*,4*R*,5*R*,6*R*)-4,5-dihydroxy-6-[(*E*,2*R*,3*S*)-3-hydroxy-2-(icosanoylamino)icos-4-enoxy]-2-(hydroxymethyl)oxan-3-yl]oxy-3-hydroxy-6-(hydroxymethyl)oxan-4-yl]oxy-4-hydroxy-6-[(1*R*,2*R*)-1,2,3-trihydroxypropyl]oxane-2-carboxylic acid. GM1 has the following structural formula:

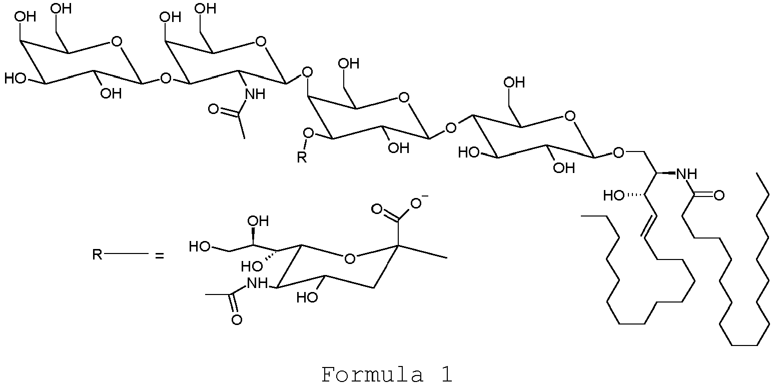

A therapeutically effective amount of monosialotetrahexosylganglioside (GM1) refers to that amount of GM1 which results in at least one of prevention or delay of onset of symptoms of PD in a subject; amelioration of symptoms of PD in a subject; prevention or delay of onset of signs of PD in a subject; amelioration of signs of PD in a subject; or a combination thereof.

As it is understood in the art, a symptom is something felt or experienced, such as pain or dizziness. As it is understood in the art, a sign is an objective observable indication of a disease, injury, or abnormal physiological state that may be detected during a physical examination, examining the patient history, or diagnostic procedure. Signs and symptoms are not mutually exclusive.

These symptoms and signs comprise unintended or uncontrollable movements, such as shaking, stiffness, and difficulty with balance and coordination and difficulty with walking and talking. Symptoms are usually assessed using the Movement Disorder Society Unified PD Rating Scale (MDS-UPDRS).

Therapeutic efficacy of PD is usually measured using the MDS-UPDRS system. The system comprises four parts: non-motor aspects of experiences of daily living are assessed in part I in a questionnaire; motor aspects of experiences of daily living are assessed in part II in a questionnaire; a motor examination is performed in part III in a clinical examination (not in a questionnaire); and motor complications from treatment are assessed in part IV in a questionnaire. The higher the MDS-UPDRS value, the more pronounced the disease.

In the context of the present invention, a dose is a measured quantity of a therapeutic agent to be taken at one time, wherein the measured quantity may be administered over a period of time.

In the context of the present invention, a therapeutically effective dose of said liposomal composition is a dose of the liposomal composition that contains an amount of GM1 which results in at least one of prevention or delay of onset of symptoms; or amelioration of symptoms of PD in a subject.

Parkinson's disease (PD) is a brain disorder that causes unintended or uncontrollable movements, such as shaking, stiffness, and difficulty with balance and coordination. Symptoms usually begin gradually and worsen over time. As the disease progresses, people may have difficulty walking and talking. They may also have mental and behavioural changes, sleep problems, depression, memory difficulties, and fatigue.

According to the invention, a dose of the liposomal composition comprising GM1 is administered at most every 4 days in a primary mode of administration with at least 3 days between each administration; preferably at most every 6 days in a primary mode of administration with at least 5 days between each administration; most preferably at most every 7 days in a primary mode of administration with at least 6 days between each administration. "At most every 7 days with at last 6 days between each administration", for example, means that if a dose is administered on a Monday, for example, the next dose of the primary mode of administration will be administered on the following Monday at the earliest.

The primary mode of administration may be any mode of administration that the person skilled in the art deems appropriate to administer the dose over a reasonable period of time.

It has been found that the liposomal composition as GM1 carrier system comprising sphingomyelin as described in this invention lead to enhanced pharmacokinetics and therapeutic properties of the encapsulated GM1.

Without wishing to be bound by theory, the liposomal composition according to the invention increases delivery of GM1 to the CNS, as the penetrability of the blood-brain barrier by the liposomal composition is higher than the penetrability by the non-liposomal GM1. Additionally, intracellular delivery of GM1 is improved as well by encapsulating it in the liposomal composition. Non-liposomal GM1 remains to a larger degree extracellular, as it has a lower penetrability of the cell membrane. The biodistribution of GM1 is thus altered in a favourable manner with the liposomal composition to increase the drug deposition in the CNS.

Furthermore and without wishing to be bound by theory, it was found that the circulation of liposomal GM1 is prolonged compared to non-liposomal GM1, as liposomal GM1 gets metabolized and excreted at a lower rate.

As a consequence of the higher penetrability of both the blood-brain barrier and the cell membrane as well as the prolonged circulation, a smaller amount of GM1 is required to obtain the desired pharmacological effect when employing the liposomal composition compared to non-liposomal GM1. This in turn means that GM1 needs to be administered less frequently.

As it has been discussed earlier, when GM1 is administered by injection, in particular by subcutaneous injection but also by other types of injection, strong pain, severe hematomas and irritation of the skin at the injection site are observed. It was found that smaller amounts of GM1 per dose and/or a lower administration frequency made possible by the liposomal composition result in a reduction of the adverse events profile in PD patients. Less severe or even the absence of pain, hematomas and skin irritation are the result.

Surprisingly, it was found that the serum half-life of the liposomal composition of the invention in venous blood plasma is longer than normally observed for non-PEGylated (PEG: polyethylene glycol) liposomal compositions comprising sphingomyelin. Non-PEGylated liposomal compositions comprising sphingomyelin for use in medical applications reported in literature have a serum half-life of up to 10 h (KRISHNA, R. et al. Liposomal and Nonliposomal Drug Pharmacokinetics after Administration of Liposome-Encapsulated Vincristine and Their Contribution to Drug Tissue Distribution Properties. JPET. 2001, Vol. 298, pages 1206 to 1212; SENIOR, J. et al. Is half-life of circulating liposomes determined by changes in their permeability? FEBS Lett. 1982, Vol. 145, No. 1, pages 109 to 114). According to the literature, one way to prolong the half-life of liposomes in blood plasma is to PEGylate the liposomes (PARK, H. et al. Evolution of drug delivery systems: From 1950 to 2020 and beyond. JCR 2022, Vol. 342, pages 53 to 65). However, the liposomal composition of the invention does not require PEGylation of the liposome and a half-life of up to 30 h is still observed. This shows that with the liposomal composition of the invention, half-lives for non-PEGylated liposomal compositions comprising sphingomyelin are achieved that the skilled person would not expect. This allows longer intervals between administrations without significant loss of therapeutic effect in the time between doses.

In particular, it was found that for the majority of PD patients treated, the liposomal GM1 composition needs to be administered at most every 7 days to achieve a desired therapeutic effect throughout the inter-dose period. Initial findings show that, in certain cases, more frequent administration (e.g. every 4 or every 6 days) is beneficial to maintain a therapeutic effect between doses. This is, however, still an improvement compared to state-of-the-art dosing regimens of twice daily for instance.

The advantages, improved efficacies and reductions in adverse events will be shown in the examples section hereinafter.

The composition is administered intravenously in the primary mode of administration. It was found that the adverse events can be mitigated not only by the smaller amount of GM1 required and the lower frequency of administration as described above, but also when the common subcutaneous injection is replaced with an intravenous injection. This results in even less pain, hematomas and skin irritation for the PD patients. In addition, intravenous administration leads to increased bioavailability compared to other forms of administration, as, for example, the gastrointestinal barrier can be bypassed.

It is preferred that the dose is administered over a period of between 45 min and 90 min, preferably about 1 h, when the composition is administered intravenously. Compared to a shorter administration period, even fewer adverse events occur when a dose is administered over this period. In the context of the present invention, the administration period of between 45 min and 90 min refers to the first dose. Even longer administration periods of between 90 min and 10 h are recommended for the second and third doses administered intravenously to avoid adverse events such as back and neck pain as well as potential pseudoallergic reactions (e.g. complement activation-related pseudoallergy (CARPA)).

In a preferred embodiment, the administration in the primary mode of administration is accompanied by administration in a secondary mode of administration in-between doses of the primary mode of administration, wherein the secondary mode of administration is preferably an oral administration. By administering additional doses in a secondary mode of administration between the doses of the primary mode of administration, the pharmacological effect is increased. For example, if the primary mode of administration is a weekly intravenous administration and the secondary mode of administration is a daily oral administration of the composition, the GM1 level in the body can be kept high. At the same time, with such an approach, the restrictions on quality of life are kept to a minimum. A subcutaneous injection of non-liposomal GM1 twice daily, as is often administered, leads to severe side effects. In comparison, an intravenous injection of liposomal GM1 once a week with additional oral doses in between, without the need for a healthcare professional to be present during oral administration, is significantly more beneficial and associated with fewer side effects for the patient. Surprisingly, despite the overall lower amount of GM1 administered and lower frequency of administration, this leads to a better pharmacological result thanks to the liposomal composition.

Preferably, the administration of the primary and/or secondary mode of administration comprises a dosing regimen of equal doses, or increasing doses of said liposomal composition comprising GM1. Possible adverse events frequently occurring in some of the first few administrations can be counterbalanced by administering smaller amounts of the liposomal composition at the earlier administrations in PD patients who experience such side effects. As soon as fewer side effects occur in a PD patient, the dose can be increased accordingly for subsequent administrations. These side effects of the liposomal composition comprise back and neck pain as well as pseudoallergic reactions (e.g. complement activation-related pseudoallergy (CARPA)) or reactions of humoral origin. It should be noted that no clinically relevant adverse events were observed with a dosing regimen corresponding to the invention.

As an alternative to administering smaller amounts of the liposomal composition to mitigate the adverse events occurring during intravenous administration, the flow rate of the infusion can be adjusted so that less liposomal composition flows into the body per unit time. Furthermore, both the amount of liposomal composition administered, and the rate of administration may be adjusted during the same administration. The choice of the amount and rate of administration depends largely on patient feedback. For example, if a patient complains of pain in the neck or/and back or/and near the injection site during administration, the amount to be administered and/or the rate of administration can be adjusted accordingly. Furthermore, the infusion can be interrupted until the symptoms of the adverse events subside.

In the context of the present invention, a dosing regimen is a schedule of doses of a therapeutic agent per unit of time including the time between doses, the amount of the therapeutic agent per dose and, in case of an intravenous injection for example, the period of time during which the dose is administered. To treat a patient, several dosing regimens of the same mode of administration can be used in succession, for example if the time between doses changes. Further, several dosage regimens can also be used in parallel, in case of different modes of administration.

Preferably, the primary mode of administration comprises a second dose of said liposomal composition comprising GM1 that is lower than the first dose and a third and subsequent doses that are increasing.

In a preferred embodiment, said composition is administered periodically every 7 days with 6 days between each administration in the primary mode of administration.

In another preferred embodiment, said therapeutically effective dose of GM1 of said liposomal composition is between 300 mg and 800 mg, preferably between 600 mg and 750 mg, most preferably about 720 mg. This dose range has been found to lead to an optimal pharmacological effect without causing severe side effects.

In a further preferred embodiment, the liposomal composition additionally comprises cholesterol, preferably sphingomyelin and cholesterol in a 1:1 molar ratio. Liposomes comprising sphingomyelin and cholesterol show an enhanced circulation lifetime and CNS bioavailability. They have improved pharmacokinetics and therapeutic characteristics. They are biocompatible and biodegradable. Although in certain cases elevated cholesterol levels may be observed after administration of the liposomal cholesterol-containing composition, these elevated cholesterol levels will return to normal levels without any resulting adverse events.

In a preferred embodiment, said therapeutically effective amount of GM1 in said liposomal composition in a single dose of the primary mode of administration is chosen such that it leads to a venous blood plasma concentration of GM1 between 50 µg/ml and 1200 µg/ml, in particular between 75 µg/ml and 600 µg/ml, further in particular between 100 µg/ml and 400 µg/ml that is reached within 1 h to 7 h, preferably within 3 h to 5 h, most preferably 4 h after the start of administration. This range of venous blood plasma concentrations of GM1 has been found to lead to an optimal pharmacological effect without causing severe side effects. It was found that with such values for a venous blood plasma concentration of GM1 that the GM1 concentration is still significantly higher compared to baseline after 96 h (see examples section hereinafter). This confirms the expectation of a long circulating drug.

In yet another preferred embodiment, the liposomes of said liposomal composition have a mean diameter between 10 nm and 70 nm, preferably between 30 nm and 70 nm, more preferably between 40 nm and 65 nm, measured by dynamic light scattering; and/or have a mean diameter between 10 nm and 50 nm, preferably between 20 nm and 50 nm, more preferably between 30 nm and 40 nm, measured by CryoTEM.

"Measured by dynamic light scattering" (DLS) means that DLS was performed on samples with a lipid concentration between 20 mg/ml and 30 mg/ml, which were diluted 1/19 in phosphate-buffered saline (PBS) or milliQ H₂O to reach an attenuation factor in the instrument of around 6. DLS was measured on a Malvern Zetasizer Nano device at 25 °C and 0° scattering angle. Instrument control and data analysis were performed with the Zetasizer software (version 7.11) from Malvern. Particle size (hydrodynamic diameter) was determined using the Stokes-Einstein equation $d \left(H\right) = \frac{kT}{3 πηD} ,$ where k is the Boltzmann constant, T is the absolute temperature, *η* is the dispersant viscosity and D is the diffusion coefficient. Viscosity was determined with the Zetasizer software and was 0.8872 cP. Dispersant refractive index was 1.330. D was obtained by fitting the autocorrelation function with a suitable algorithm. Cumulants analysis is a simple method of analysing the autocorrelation function generated by a DLS experiment and produces the mean particle size (Z-ave) and polydispersity index (PDI). The calculation is defined in ISO 13321 (1996) and ISO 22412 (2008). The first order result from a DLS experiment is an intensity distribution of particle sizes. The intensity distribution is naturally weighted according to the scattering intensity. The size distribution is displayed as a plot of the relative intensity of light scattered by particles (on the Y axis) versus various size classes (on the X axis) which are logarithmically spaced. Clear disposable zeta cells with a pathlength of 10 mm were used for the measurements. Usually but not necessarily, the liposomes in the inventive composition will fall into the numerical ranges of size measured by the method described.

"Measured by CryoTEM" means that the samples were subject to cryogenic transmission electron microscopy (CryoTEM). The liposomal samples diluted as appropriate, vitrified and prepared on-grid (Formvar and Carbon) with an acceleration voltage of 200 kV. Images were acquired with a CryoTEM JEOL JEM-2100F a TVIPS TemCam F415MP camera at 20'000x; 40'000x; 80'000x magnification. Particle identification and size determination were performed by semi-automated image processing using Vironova Analyzer Software, Vironova, Sweden. Briefly, a series of random images of the same magnification was imported. Only liposome particles located entirely within the boundaries of the image and with a distinct membrane were detected. The identified objects were analysed for spherical diameter, circularity, unilamellar-ity. All images were batch-processed with identical thresholds and settings, accumulating over 5 to 18 images for each sample, corresponding 6 to a number of particle analysed of 1560 to 1178. Mean values have a standard deviation of approx. 10 nm.

Usually but not necessarily, the liposomes in the inventive formulation will fall into the numerical ranges of size measured by both methods. The diameter size measured by CryoTEM is generally lower than the diameter size measured by DLS.

In the context of the present invention, the phrase "and/or" in relation to the measurement of the liposomal diameter is to be understood as an inclusive disjunction. This means that all liposomal compositions are included in which the liposomes usually but not necessarily fulfil either or both conditions.

It has successfully been shown that the inventive liposomes of said composition and the indicated size are more stable than those known in the art. Liposomes of such small diameter are op-sonized less rapidly and at a lower extent than their larger counterparts and are cleared less rapidly by the reticuloendo-thelial system. Also, larger liposomes are more likely to fuse or interact with other liposomes or particles.

As a result, the serum half-life in humans of the liposomal composition according to the invention is considerably higher than the one for liposomal compositions with higher liposome diameters known in the art. Additionally, a smaller liposomal diameter allows a facilitated crossing of the blood-brain barrier. The composition thus provides higher drug exposition for a given dose in the CNS (HERSH, A.M. Crossing the Blood-Brain Barrier: Advances in Nanoparticle Technology for Drug Delivery in Neuro-Oncology. Int. J. Mol. Sci. 2022, Vol. 23, pages 1 to 28).

In a preferred embodiment, the liposomal composition comprises phosphate-buffered saline (PBS) at a pH of about 6.8, which corresponds to a physiologically well-tolerated pH.

In another preferred embodiment, the liposomal composition comprises at least one of a pharmaceutically acceptable additive, a carrier, an excipient and a diluent.

In the context of the present invention, an additive is a substance which is added in the composition along the API so as to impart specific qualities in the composition. An additive has very little or no therapeutic value but is necessary in the manufacture of a particular form of administration. An additive may serve any one of the following purposes or any combination thereof: provide bulk to the composition; facilitate drug absorption or solubility and other pharmacokinetic considerations; aid in handling of the API during manufacturing; provide stability and prevent from denaturation.

In the context of the present invention, a carrier (also known as drug carrier or drug vehicle) is a substrate used in the process of drug delivery which serves to improve the selectivity, effectiveness and/or safety of drug administration. Popular types of carriers include liposomes, micelles, microspheres and nanoparticles.

In the context of the present invention, an excipient is a substance formulated alongside the API, included for the purpose of long-term stabilization, bulking up solid formulations that contain potent active ingredients in small amounts, or to confer a therapeutic enhancement on the API, such as facilitating drug absorption, reducing viscosity or enhancing solubility or enhancing bioavailability.

In the context of the present invention, a diluent (also referred to as a filler, dilutant or thinner) is an ingredient in a medicinal preparation that lacks pharmacologic activity but is pharmaceutically necessary or desirable. It is particularly useful in increasing the bulk of potent drug substances with a mass too small for dosage to allow manufacture or administration.

In a preferred embodiment, the method of treating PD is selected from reducing tremor, increasing physical movement, increasing walking speed, improving walking ability, improving motion control, improvement of speech changes, in a patient with PD. The above complaints are common symptoms in PD patients. By treating the affected areas of the CNS with the liposomal composition according to the invention, it was found that these symptoms could be significantly reduced, when assessing the severity of the symptoms using the MDS-UPDRS system.

Another aspect of the present invention relates to a liposomal composition for use in a method of treating Parkinson's disease (PD), said liposomal composition comprising sphingomyelin in a lipid bilayer and a therapeutically effective amount of monosialotetrahexosylganglioside (GM1), wherein the treatment is an at least partial reversal of PD, and wherein the treatment optionally involves a dosing regimen with a therapeutically effective dose of said liposomal composition being administered in a primary mode of administration at most every 4 days with at least 3 days between each administration; preferably at most every 6 days with at least 5 days between each administration; most preferably at most every 7 days with at least 6 days between each administration.

The monosialotetrahexosylganglioside (GM1) is partially located in a membrane and partially located in an aqueous core of liposomes. The composition is administered intravenously in the primary mode of administration.

In the context of the present invention, an at least partial reversal of PD is an at least partial decrease in at least one of symptoms or signs of the disease, wherein at least one of symptoms or signs do not increase substantially, compared to baseline.

It was surprisingly found that treatment of PD patients with the liposomal composition of the invention over a period of 24 weeks does not merely halt the negative development of PD, but that the symptoms and signs can be significantly reduced, even in cases where the liposomal composition of the invention is administered at 7-day intervals.

The invention will be further explained by the following examples. The examples are not intended to limit the scope of the invention in any way.

The preparation of the liposomal composition containing GM1 was described in the prior art (WO 2019122220 A1 (INNOMEDICA HOLDING AG) 27.06.2019). In particular, the circularity of the liposomes used for the liposomal composition of the present invention is greater than or equal to 0.95, in particular 0.98-1.00.

The liposomal composition comprising GM1 used for the intravenous administration in the following examples may hereinafter be called "TLN" or "Talineuren".

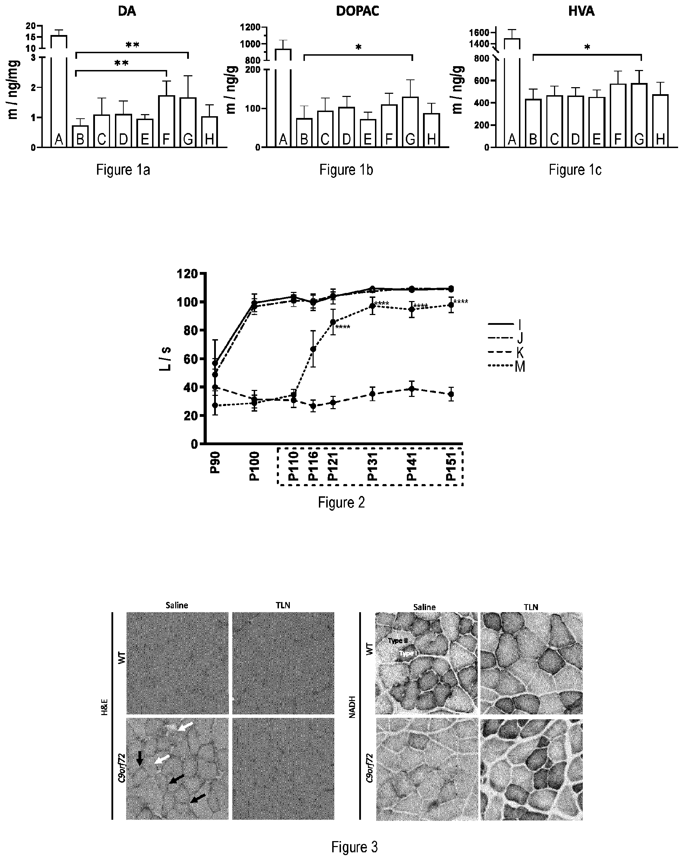

- Figure 1a-1c:: striatal levels of dopamine and its metabolites in mice of the MPTP-PD model: levels of dopamine (DA, Figure 1a), 3,2-dihydroxyphenylacetic acid (DOPAC, Figure 1b) and homovanillic acid (HVA, Figure 1c) determined by HPLC, n=5-10, bars indicate means and standard deviation, one-way ANOVA treatment groups p-value *<0.05, **<0.01;
- Figure 2:: reverse rotarod test performance of *C9orf72* mice: reverse rotarod test with treatment initiation at P110 until P151 (15 mg/kg saline or Talineuren (TLN), intravenous administration, every other day, dotted box), n=5-7/grp. Values represent means and standard error of means. Differences were assessed using 2way ANOVA with Bonferroni's multiple comparisons test, ****p < 0.0001;
- Figure 3:: hematoxylin and eosin (H&E) and NADH staining of the gastrocnemius muscle of *C9orf72* mice at P170. Left: H&E staining of the gastrocnemius muscle. The black arrows show angular fibres. The white arrows indicate muscle fibres with a central nucleus indicative of denervation. Right: NADH staining showing Type I (dark) and Type II (light) myofibers. n = 3 animals/grp. Samples were stained and imaged with the same settings;

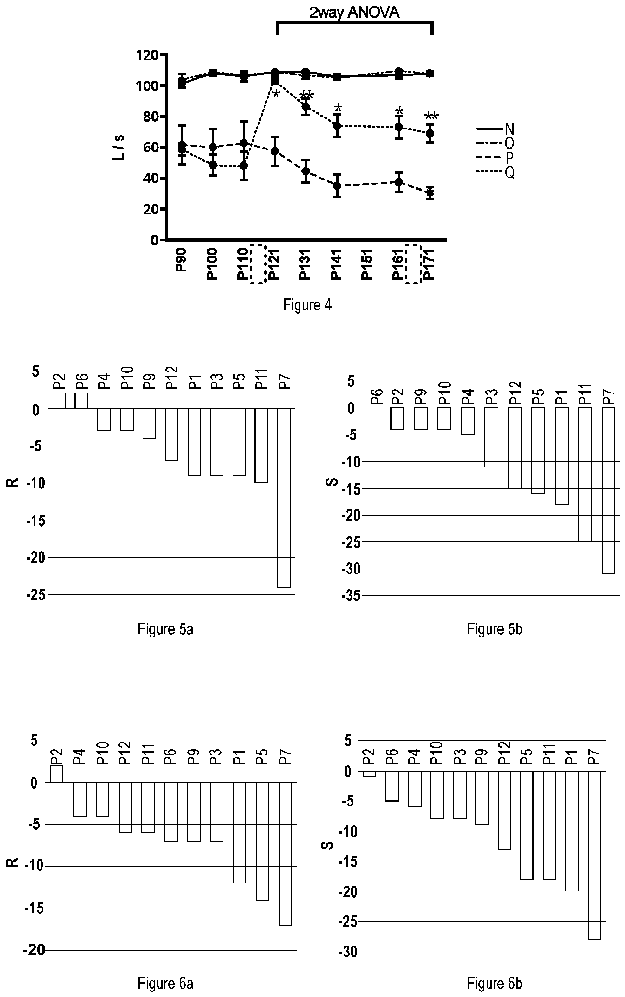

- Figure 4:: reverse rotarod test performance of *SOD1-G93A* mice, with treatment initiation at P110 to P121 and P160 to P170 (15 mg/kg saline or Talineuren ganglioside (TLN), intravenous administration, every other day, dotted boxes), n=4-6/grp. Values represent means and standard error of means. Differences were assessed using 2way ANOVA with Bon-ferroni's multiple comparisons test, ****p < 0.0001;
- Figure 5a+5b:: difference in MDS-UPDRS motor (Figure 5a) and total (Figure 5b) score for each patient (P1-P7; P9-P12) between baseline and assessment after 8 weeks of treatment. 5a: change in UPDRS motor score ("off" standard medication for 12 h) (R); 5b: change in UPDRS total score (S);
- Figure 6a+6b:: difference in MDS-UPDRS motor (Figure 6a) and total (Figure 6b) score for each patient (P1-P7; P9-P12) between baseline and assessment after 24 weeks. 6a: change in UPDRS motor score ("off" standard medication for 12 h) (R); 6b: change in UPDRS total score (S);

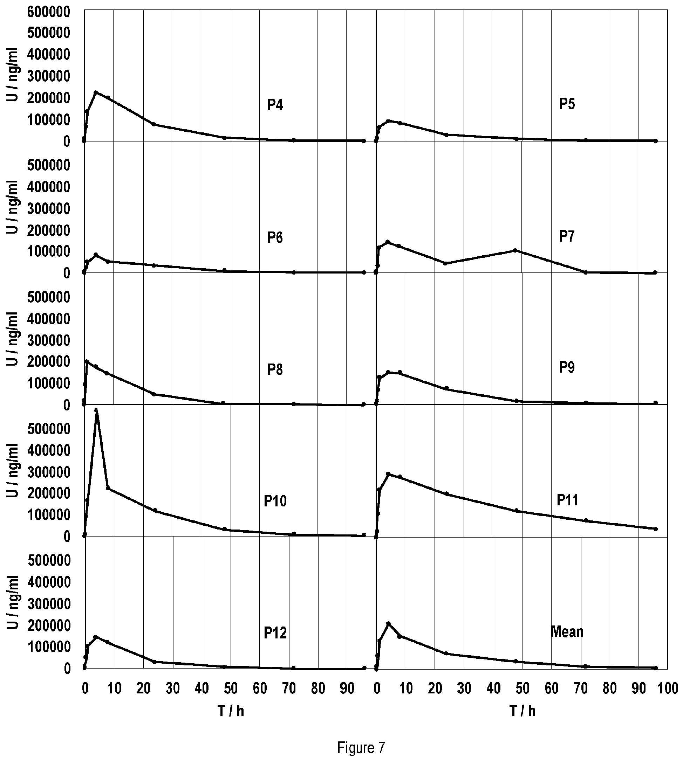

- Figure 7:: GM1 concentration (U) over time (T) in the blood plasma for all nine patients in the dose consolidation group and the mean value of each measured point in time;

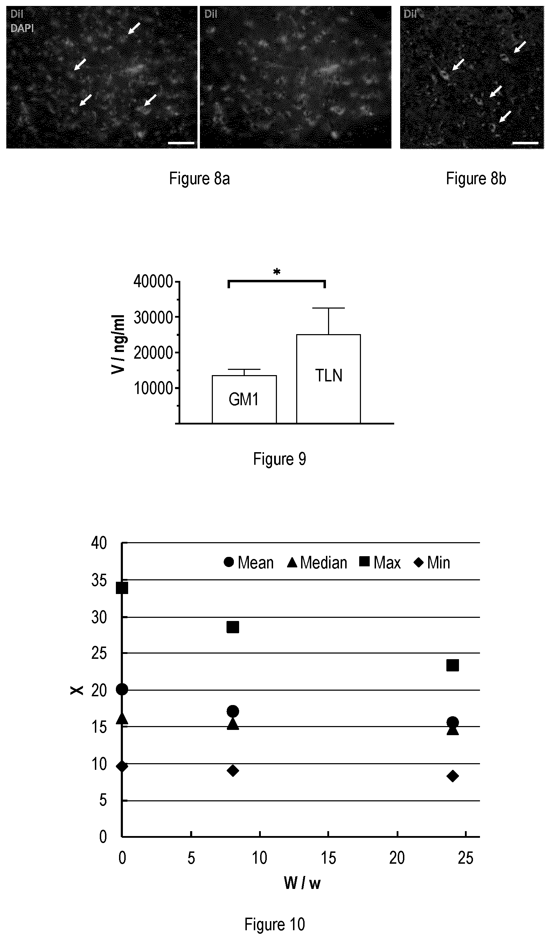

- Figure 8a+8b:: TLN-DiI uptake in cells of the mouse brain: 8a: substantia nigra after 48 h (TLN-DiI, i.v., 30 mg/kg). DAPI (nuclear DNA, blue), DiI (dye, red). 8b: motor cortex after 24 h (TLN-DiI, i.v., 15 mg/kg) Scale bar = 50 µm, white arrows indicate DiI-positive cells;
- Figure 9:: GM1 levels in the rat brain (V) determined by LC-MS/MS after 12.3 mg/kg bodyweight administration of GM1 (free or in form of Talineuren) intravenously once daily. (t-test with welch correction, p-value < 0.05), bars indicate mean and standard deviation, n = 5;
- Figure 10:: Change in Parkinson's Disease Questionnaire (PDQ-39) mean score of the eight areas of daily living (of which the mean, median, max, and min value is shown from patients P1 to P12) (X) over time at baseline (time t = 0 weeks (w)), after 8 weeks and after 24 weeks of treatment; W: time in weeks (w).

Talineuren has been investigated in a number of preclinical models of neurological diseases. Efficacy studies have been conducted in animal models of different movement disorders, such as the MPTP-mouse model of PD, the *C9orf72* and *Sod1* mouse model for amyotrophic lateral sclerosis.

### Example 1: Talineuren in models of PD

MPTP (1-methyl-4-phenyl-1,2,3,6-tetrahydrophyridine) is a prodrug to the neurotoxin MPP⁺, which causes permanent symptoms of PD by destroying dopaminergic neurons in the substantia nigra of the brain.

Studies in the MPTP-mouse model of PD, where the dopaminergic neurodegeneration is induced by a neurotoxin produced mixed results. MPTP was given twice a day at the dose of 20 mg/kg in saline i.p. (intraperitonial) at 4 h intervals on two consecutive days (Days 1 and 2), the total amount being then 80 mg/kg. Dosing volume for MPTP was 10 mL/kg and pure MPTP active compound concentration was 1.6 mg/mL (after salt correction factor had been reduced). MPTP dosing was started in the morning (8-9 AM).

Mice received Talineuren, p.o. (per os; orally) daily for 14 days (Days 1-14). The dosing volume was 5 ml/kg. On MPTP dosing days (Days 1-2) the compound dosing was performed on the evening (6-8 PM). On Day 3 the compound dosing was performed on afternoon (1-2 PM). On Days 4-14 the compound dosing was performed starting 7 AM.

Figure 1 shows the striatal levels of dopamine (DA, Figure 1a), 3,2-dihydroxyphenylacetic acid (DOPAC, Figure 1b) and homovanillic acid (HVA, Figure 1c) determined by high-performance liquid chromatography (HPLC). 5-10 subjects were examined for each treatment group and the bars indicate means and standard deviation in the wet tissue. The groups received the following treatments:

| | |
|---|---|
| A: | Sham; |
| B: | MPTP + PBS; |
| C: | MPTP + BV (base vesicle) 30 mg/kg, p.o.; |
| D: | MPTP + GM1 30 mg/kg, i.p.; |
| E: | MPTP + GM1 30 mg/kg, p.o.; |
| F: | MPTP + TLN 7.5 mg/kg, p.o.; |
| G: | MPTP + TLN 15 mg/kg, p.o.; and |
| H: | MPTP + TLN 30 mg/kg, p.o. |

In the first study with oral administration of 7.5 mg/kg (F) and 15 mg/kg (G) Talineuren showed partial rescue of dopamine and dopamine metabolite levels in the substantia nigra.

### Example 2: Talineuren in Models of ALS

Amyotrophic lateral sclerosis (ALS) is a progressive nervous system disease that affects nerve cells in the brain and spinal cord, causing loss of muscle control.

To evaluate a potential therapeutic effect in ALS, two preclinical models with the most frequent mutations in familial ALS were used. In the *C9orf72* and *SOD1* mouse model for amyotrophic lateral sclerosis Talineuren ganglioside improved motor performance of mice and reduced ER stress on a cellular level. The liposomes uptake was shown on in vitro cultures of cortical neurons in culture but also in motoneurons of the spinal cord in vivo. More importantly, Talineuren ganglioside treatment in the *C9orf72* transgene model of ALS showed a remarkable effect on motor performance. Motor function and coordination were significantly improved not only upon intravenous Talineuren treatment initiation at an early stage of the disease but also when started at a more advanced stage.

Figure 2 shows the reverse rotarod test performance of *C9orf72* mice. The test is used to assess motor coordination and balance in rodents. The apparatus consists of a motorized, circular rod turning at a constant or increasing speed. Rodents naturally try to stay on the rotarod to avoid falling on the platform. The latency to fall (L) is measured in seconds. Figure 2 shows the following curves:

| | |
|---|---|
| I: | wildtype mice injected with saline only; |
| J: | wildtype mice injected with Talineuren; |
| K: | *C9orf72*-mice injected with saline only; |
| M: | *C9orf72*-mice injected with Talineuren. |

The treatment was initiated at postnatal day 110 (P110) until P151 (dotted box in Figure 2). When intravenous treatment was initiated at the early stage of the disease and continued, prolonged latency to fall (L) at nearly wildtype (WT) level was observed in the *C9orf72*-mice injected with Talineuren (M). The high latency to fall (L) was sustained until the end of treatment at P151. Throughout the treatment period, this effect remained highly significant. In the hanging wire test performed at postnatal day 140 (P140) muscular strength was also improved in the *C9orf72* mice treated with Talineuren ganglioside compared to the *C9orf72* saline group.

These changes in the physical condition of the mice were supported by microscopic observation on sections of the spinal cord (Figure 3). Talineuren ganglioside treatment mitigated metabolic stress as shown by downregulation of markers for Endoplasmic Reticulum stress (BiP: glucose-regulated protein), which is known to be involved in ALS progression.

Neuronal inclusions of PolyGA, a protein translated from the repeat expansion in the *C9orf72* mutation, are abundant in patients with ALS and detected in post-mortem tissues. PolyGA aggregation is correlated with the unfolded protein response and triggers behavioural deficits through inflammation and protein sequestration that likely contribute to the prodromal symptoms and disease progression of *C9orf72* patients (SCHLUDI, M.H. et al. Spinal poly-GA inclusions in a C9orf72 mouse model trigger motor deficits and inflammation without neuron loss. Acta Neuropathol. 2017, Vol. 134, No. 2, pages 241 to 254). In the Talineuren-treated animals, there is a significant reduction of the large PolyGA aggregates. In the skeletal muscle, denervation of the neuromuscular junctions of mice carrying the *C9orf72* mutation has been previously described (LIU, Y. et al. C9orf72 BAC Mouse Model with Motor Deficits and Neurodegenerative Features of ALS/FTD. Neuron 2016, Vol. 90, No. 3, pages 521 to 534). In preclinical testing, H&E staining of the gastrocnemius muscle at P170 revealed groups of angular fibres and muscle fibres with central nucleus indicative of muscle fibre degeneration and denervation in *C9orf72* untreated mice but not in WT mice or *C9orf72* mice treated with Talineuren (see panels on the left of Figure 3). In additional, nicotinamide adenine dinucleotide (NADH) staining showed Type I and II myofibers in WT mice. The myofibrillar architecture was reduced in untreated *C9orf72* mice, indicating mitochondrial or sarcoplasmic reticulum abnormalities and neurogenic process (see panels on the right of Figure 3). *C9orf72* animals that received Talineuren showed almost WT characteristics.

The therapeutic effect of intravenously administered Talineuren was also observed in the preclinical ALS model with the SOD1 mutation. Figure 4 shows the following curves:

| | |
|---|---|
| N: | wildtype mice injected with saline only; |
| O: | wildtype mice injected with Talineuren; |
| P: | *C9orf72*-mice injected with saline only; |
| Q: | *C9orf72*-mice injected with Talineuren. |

Two treatment intervals between P110 and P121 as well as between P161 and P171 are marked with dotted boxes in Figure 4. Ten days of treatment starting at postnatal day P110 resulted in improved rotarod performance of the animals carrying the mutation. Although the effect was diminished over time, animals that received the treatment sustained improved performance. A second treatment interval at P160 could not enhance performance, however.

### Example 3: Clinical Trial Results

A phase I clinical trial has been conducted as an open-label, single ascending dose escalation (n = 3) followed by a multiple administration dose at the maximal suitable dose (n = 9) (Neu-rologisches Institut Konolfingen; ClinicalTrials.gov identifier: NCT04976127). The primary objective was to demonstrate the safety of TLN administration intravenously in Parkinson's patients. Secondary objectives were the determination of the maximal suitable dose based on the safety profile and preliminary efficacy, as well as the determination of the pharmacokinetics profile.

### Dose Escalation

The administered dose in all three PD patients was increased on a weekly basis up to the maximal intended dose of 720 mg without any serious adverse events nor dose delays or dose modifications. Thus, the maximal suitable dose for the dose consolidation part of the trial was determined at the highest dose-level (720 mg). This confirms the expected good tolerability of the drug.

### Dose Consolidation

Nine additional patients were enrolled in the trial and treated at this established therapy dose for a period of eight weeks. All 9 patients have completed the Talineuren treatment with a duration of 8 weeks. Pharmacokinetic blood samples measuring GM1 in serum were taken at 10 different time points during the first day of Talineuren administration and the following days. All samples were analysed and the results are presented below.

### Optional prolongation of treatment

All patients already enrolled into the trial expressed the wish to continue with weekly Talineuren treatment, as they subjectively experienced benefits from the treatment. As Talineuren is generally well tolerated, the trial was extended twice (prolongation 1: 8 weeks; prolongation 2: 16 weeks). This allowed the continuation of an eventual benefit from Talineuren for the patients whilst gathering further safety data on the investigational medicinal product from repeated dosing with 720 mg.

### Safety / Tolerability

Treatment is generally well tolerated and no serious adverse events have occurred. Several patients experienced CARPA (complement-activation related pseudoallergy; patients experienced neck pain and headache that rapidly stopped with the reduction of the speed of the infusion) which are according to the safety definitions of the Clinical Trial Protocol not judged as serious adverse events. CARPA is a known phenomenon in nanotechnological investigational medicinal products, and all CARPAs experienced in this study were fully reversible.

### Efficacy After 8 Weeks of Treatment

The efficacy analysis after 8 weeks of treatment contains data from all patients that were treated for the full study period of 8 weeks of treatment with weekly infusions of 720 mg of the active pharmaceutical ingredient GM1 ganglioside in the liposomal formulation of the invention (11 out of 12 patients). One patient (Patient no. 8) only received the treatment for two weeks and dropped out of the study.

For efficacy measurement, two evaluations were considered:
1) The MDS-UPDRS motor score (part III). Following the literature (SCHNEIDER, J.S. et al. A Randomized, Controlled, Delayed Start Trial of GM1 Ganglioside in Treated Parkinson's Disease Patients. J. Neurol. Sci. 2013, Vol. 324, No. 1, pages 140 to 148), the evaluation was performed in an "off" medication state, meaning that the patients were put off their standard treatment for 12 hours. The evaluation was performed at two timepoints: the first being the baseline (BL) measurement before treatment start, the second being one week after the final treatment during the final assessment (FA) after 8 weeks.
2) The MDS-UPDRS total score. Part III was measured as described in 1). Parts I, II and IV correspond to the standard evaluation procedure of MDS-UPDRS using questionnaires. The evaluations were performed during the first Talineuren treatment (BL) and one week after the last treatment (FA).

Table 1 shows the data points for each patient separately according to the trial database for both considered efficacy measures (motor score in the "off" state and MDS-UPDRS total score). Columns three and six show the difference between the baseline (BL) assessment and the final assessment (FA) after 8 weeks for each patient. The motor score was on average reduced by 6.7 points and the total score by 12.2 points, respectively. Using a Wilcoxon rank sign test for non-parametric, paired samples, the differences are statistically significant at the 1 % level. Clinically, the difference is also considered relevant since the "minimally clinically important difference" (MCID) according to literature is an improvement by about 4.3 to 5 points.

**Table 1**

| | Part III: Motor Score | | | MDS-UPDRS Total Score | | |
|---|---|---|---|---|---|---|
| | BL off | FA off | Difference | Total BL | Total FA | Difference |
| Patient 1 | 17 | 8 | **-9** | 36 | 18 | **-18** |
| Patient 2 | 8 | 10 | **2** | 16 | 12 | **-4** |
| Patient 3 | 10 | 1 | **-9** | 25 | 13 | **-12** |
| Patient 4 | 6 | 3 | **-3** | 10 | 5 | **-5** |
| Patient 5 | 17 | 8 | **-9** | 29 | 13 | **-16** |
| Patient 6 | 17 | 19 | **2** | 51 | 51 | **0** |
| Patient 7 | 36 | 12 | **-24** | 88 | 57 | **-31** |
| Patient 9 | 22 | 18 | **-4** | 28 | 24 | **-4** |
| Patient 10 | 9 | 6 | **-3** | 19 | 15 | **-4** |
| Patient 11 | 17 | 7 | **-10** | 41 | 16 | **-25** |
| Patient 12 | 18 | 11 | **-7** | 32 | 17 | **-15** |
| Mean | 16.1 | 9.4 | **-6.7** | 34.1 | 21.9 | **-12.2** |

Figure 5a and Figure 5b show the difference between the baseline (BL) and the final assessment (FA) after 8 weeks of the MDS-UPDRS motor score (part III) and the MDS-UPDRS total score, respectively, for each patient (P1-P7;P9-P12) in descending order. The labels on the Y-axis mean:

| | |
|---|---|
| R: | change in UPDRS motor score ("off" standard medication for 12 h); |
| S: | change in UPDRS total score. |

### Efficacy After 24 Weeks of Treatment

Due to the promising results after 8 weeks of treatment, the treatment was continued up to a total treatment period of 24 weeks. The efficacy analysis after 24 weeks of treatment contains data from patients P1-P7 and P9-P12 that received weekly infusions of 720 mg of the active pharmaceutical ingredient GM1 ganglioside in the liposomal formulation of the invention.

**Table 2**

| | Part III: Motor Score | | | MDS-UPDRS Total Score | | |
|---|---|---|---|---|---|---|
| | BL off | FA off | Difference | Total BL | Total FA | Difference |
| Patient 1 | 17 | 5 | **-12** | 36 | 16 | **-20** |
| Patient 2 | 8 | 10 | **2** | 16 | 15 | **-1** |
| Patient 3 | 10 | 3 | **-7** | 25 | 17 | **-8** |
| Patient 4 | 6 | 2 | **-4** | 10 | 4 | **-6** |
| Patient 5 | 17 | 3 | **-14** | 29 | 11 | **-18** |
| Patient 6 | 17 | 10 | **-7** | 51 | 46 | **-5** |
| Patient 7 | 36 | 19 | **-17** | 88 | 60 | **-28** |
| Patient 9 | 22 | 15 | **-7** | 28 | 19 | **-9** |
| Patient 10 | 9 | 5 | **-4** | 19 | 11 | **-8** |
| Patient 11 | 17 | 11 | **-6** | 41 | 23 | **-18** |
| Patient 12 | 18 | 12 | **-6** | 32 | 19 | **-13** |
| Mean | 16.1 | 8.6 | **-7.5** | 34.1 | 21.9 | **-12.2** |

Table 2 shows the data points for patients P1-P7 and P9-P12 for both considered efficacy measures (motor score in the "off" state and MDS-UPDRS total score). Columns three and six show the difference between the baseline (BL) assessment and the final assessment (FA) after 24 weeks for each patient. The motor score was on average reduced by 7.5 points and the total score by 12.2 points, respectively. These results indicate that there is a further improvement in the MDS-UPDRS motor score (part III) after 24 weeks of total treatment when compared to the results after 8 weeks of total treatment.

Figure 6a and Figure 6b show the difference between the baseline (BL) and the final assessment (FA) after 24 weeks of the MDS-UPDRS motor score (part III) and the MDS-UPDRS total score, respectively, for each patient (P1-P7; P9-P12) in descending order. The labels on the Y-axis mean:

| | |
|---|---|
| R: | change in UPDRS motor score ("off" standard medication for 12 h); |
| S: | change in UPDRS total score. |

### Efficacy After 56 Weeks of Treatment

Analogously to the efficacy analysis after 24 weeks of treatment, the treatment was continued for a total of 56 weeks for the same patients, except for patient P11. Again, the patients received weekly infusions of 720 mg of the active pharmaceutical ingredient GM1 ganglioside in the liposomal formulation of the invention.

**Table 3**

| | Part III: Motor Score | | | MDS-UPDRS Total Score | | |
|---|---|---|---|---|---|---|
| | BL off | FA off | Difference | Total BL | Total FA | Difference |
| Patient 1 | 17 | 8 | **-9** | 36 | 19 | **-17** |
| Patient 2 | 8 | 10 | **2** | 16 | 16 | **0** |
| Patient 3 | 10 | 3 | **-7** | 25 | 22 | **-3** |
| Patient 4 | 6 | 1 | **-5** | 10 | 3 | **-7** |
| Patient 5 | 17 | 5 | **-12** | 29 | 15 | **-14** |
| Patient 6 | 17 | 10 | **-7** | 51 | 42 | **-9** |
| Patient 7 | 36 | 17 | **-19** | 88 | 57 | **-31** |
| Patient 9 | 22 | 17 | **-5** | 28 | 20 | **-8** |
| Patient 10 | 9 | 7 | **-2** | 19 | 12 | **-7** |
| Patient 11 | 17 | N/A | **N/A** | 41 | N/A | **N/A** |
| Patient 12 | 18 | 10 | **-8** | 32 | 20 | **-12** |
| Mean | 16.1 | 8.8 | **-7.3** | 34.1 | 22.6 | **-11.5** |

The data in Table 3 show that the values remain at a low level compared to the baseline values even after 56 weeks.

### Parkinson's Disease Questionnaire (PDQ-39)

In addition to the values used to determine the severity of Parkinson's disease, which were determined using the Unified Parkinson's Disease Rating Scale (UPDRS), the effectiveness of the treatment was also analysed using the Parkinson's Disease Questionnaire (PDQ-39). This questionnaire, comprising 39 questions, assesses how often people with Parkinson's disease experience difficulties across eight areas (dimensions) of daily living: mobility, activities of daily living (ADL), emotional well-being, stigma, social support, cognition, communication, bodily discomfort. Each area's total score ranges from 0 (the patient never has difficulties) to 100 (the patient always has difficulties).

The questionnaire was carried out with the above-mentioned 12 patients P1-P12 at baseline (t = 0), after 8 weeks of treatment and after 24 weeks of treatment (patient P8 did not complete the questionnaire after 24 weeks). At each time point (W, t = 0 w, 8 w, and 24 w), the mean value from the eight areas of daily living was calculated for each patient. From the resulting values of the 12 patients, the mean, median, max and min values were calculated for each time point.

Figure 10 shows these values (X) over time (W; in weeks (w)). The mean value (circle) shows a reduction in the PDQ-39 score from 20.1 at time 0, to a score of 17.2 after 8 weeks, to a score of 15.6 after 24 weeks. The median (triangle), max (square) and min (diamond) values show the same trend over time. A particularly strong improvement was observed in the patient with the initially most severe score (patient 6: 33.9). The max values after 8 weeks (28.7) and after 24 weeks (23.3) both belong to this patient.

These results show that in patients treated with the liposomal composition and dose regimen according to the invention, a significant improvement is observed not only in the MDS-UPDRS assessment procedure, but also in a second assessment procedure (the PDQ-39 scoring system).

### Pharmacokinetics / Pharmacodynamics

The samples of the 9 patients with weekly infusion of 720 mg Talineuren (dose consolidation part) were collected during 10 timepoints during the first infusion (0 min, 5 min, 30 min, 1 h, 4 h, 8 h, 24 h, 48 h, 72 h, 96 h).

**Table 4**

| Patient | tₘₐₓ h | Cₘₐₓ ng/mL | t_{1/2} h |
|---|---|---|---|
| 4 | 4 | 225000 | 11.3 |
| 5 | 4 | 91900 | 12.6 |
| 6 | 4 | 83900 | 19.1 |
| 7 | 4 | 144000 | 10.9 |
| 8 | 1 | 199000 | 10.2 |
| 9 | 4 | 147000 | 13.2 |
| 10 | 4 | 582000 | 14.8 |
| 11 | 4 | 290000 | 30.7 |
| 12 | 4 | 146000 | 11.9 |
| N | 9 | 9 | 9 |
| Mean | | 212000 | 15.0 |
| SD | | 153000 | 6.46 |
| Min | 1 | 83900 | 10.2 |
| Median | 4 | 147000 | 12.6 |
| Max | 4 | 582000 | 30.7 |
| Coeff. of variance CV% | | 72.2 | 43.2 |
| Geometric mean | | 178000 | 14.1 |
| CV% geom. mean | | 65.2 | 35.8 |

Table 4 shows the time at which the highest blood plasma concentration was reached (tₘₐₓ), the highest blood plasma concentration (cₘₐₓ) and the half-life (t_{1/2}) of GM1 in the blood plasma for each of the nine patients. Further, the mean, the standard deviation (SD), the minimum, the median, the maximum, the coefficient of variance (CV%), the geometric mean and the geometric mean of the coefficient of variance of these values are shown. Figure 7 shows the GM1 concentration over time in the blood plasma for all nine patients and the mean value of each measured point in time. The labels on the axes mean:

| | |
|---|---|
| T: | time after infusion start in h; |
| U: | GM1 concentration in ng/ml. |

GM1 was measured in all samples. Most patients have reached their highest venous blood plasma concentration after 4 h. The infusion lasted 90 min and there was no sampling at that timepoint. Therefore, the highest blood plasma concentration might also be reached earlier. There is still a significantly higher level of GM1 after 96 h compared to the baseline, which confirms the expectation of a long circulating drug.

The mean of the half-life is slightly lower than expected, although there are high individual numbers (patient 11) and a broad standard deviation. There might be no accumulation in the blood plasma with the currently measured mean half-life and a frequency of just one dose per week. It might therefore be interesting to test different dose-frequencies in order to reach a steady-state condition. The mean of the volume of distribution is relatively low and might suggest that GM1 is mostly in the blood. It might also mean, that GM1 is widely distributed to the central nervous system and not to other hydrophobic parts of the body.

### Example 4: Brain Penetrance

Talineuren delivery to the brain has been assessed in mice. Flu-orescently labelled Talineuren (TLN-DiI) was administered to mice and accumulation of the dye was determined 24 h or 48 h later.

Figure 8a shows TLN-DiI uptake in cells of the substantia nigra of the mouse brain after 48 h (TLN-DiI, i.v., 30 mg/kg). The left image of Figure 8a shows both the nuclear DNA (DAPI, blue) and the DiI dye (red), the right image of Figure 8a shows the DiI dye (red) only. Figure 8b shows TLN-DiI uptake in cells of the motor cortex of the brain after 24 h (TLN-DiI, i.v., 15 mg/kg). The DiI signal was detected in cells of substantia nigra as well as the motor cortex (white arrows).

The accumulation of Talineuren's active pharmaceutical ingredient (GM1) in the rat brain was investigated in a study with CD-1 rats. Each animal received a dose of 12.3 mg/kg body weight of either free (GM1) or liposomal GM1 (TLN) intravenously once per day for 4 consecutive days. GM1 levels were quantified in the brain 24 h after the last dose by liquid chromatography-mass spectrometry/mass spectrometry (LC-MS/MS).

Figure 9 shows the GM1 levels in the rat brain (V) after four days of treatment. The values were significantly increased in the group that received Talineuren (liposomal GM1; TLN) compared to the group receiving free GM1. This indicates that GM1 is more efficiently delivered to the brain when inserted into the liposome.

## Claims

1. A liposomal composition for use in a method of treating Parkinson's disease (PD), said liposomal composition comprising sphingomyelin in a lipid bilayer and a therapeutically effective amount of monosialotetrahexosylganglioside (GM1), wherein the monosialotetrahexosylganglioside (GM1) is partially located in a membrane and partially located in an aqueous core of liposomes,
**characterised in that** a therapeutically effective dose of said liposomal composition is administered at most every 4 days in a primary mode of administration with at least 3 days between each administration; preferably at most every 6 days in a primary mode of administration with at least 5 days between each administration; most preferably at most every 7 days in a primary mode of administration with at least 6 days between each administration;
wherein the composition is administered intravenously in the primary mode of administration.

2. The composition for use according to claim 1, wherein the dose is administered over a period of between 45 minutes and 90 minutes, preferably about 1 hour.

3. The composition for use according to any one of the preceding claims, wherein the administration in the primary mode of administration is accompanied by administration in a secondary mode of administration in-between doses of the primary mode of administration, wherein the secondary mode of administration is preferably an oral administration.

4. The composition for use according to any one of the preceding claims, wherein the administration of the primary and/or secondary mode of administration comprises a dosing regimen of equal doses, or increasing doses of said liposomal composition comprising GM1.

5. The composition for use according to any one of claims 1 to 3, wherein the primary mode of administration comprises a second dose of said liposomal composition comprising GM1 that is lower than the first dose and/or the second dose is administered at a lower flow rate than the first dose.

6. The composition for use according to any one of the preceding claims, wherein said composition is administered periodically every 7 days with 6 days between each administration in the primary mode of administration.

7. The composition for use according to any one of the preceding claims, wherein said therapeutically effective dose of GM1 of said liposomal composition is between 300 mg and 800 mg, preferably between 600 mg and 750 mg, most preferably about 720 mg.

8. The composition for use according to any one of the preceding claims, wherein the liposomal composition additionally comprises cholesterol, preferably sphingomyelin and cholesterol in a 1:1 molar ratio.

9. The composition for use according to any one of the preceding claims, wherein the liposomes of said liposomal composition
- have a mean diameter between 10 nm and 70 nm, preferably between 30 nm and 70 nm, more preferably between 40 nm and 65 nm, measured by dynamic light scattering; and/or
- have a mean diameter between 10 nm and 50 nm, preferably between 20 nm and 50 nm, more preferably between 30 nm and 40 nm, measured by CryoTEM.

10. The composition for use according to any one of the preceding claims, wherein the liposomal composition comprises phosphate buffer saline at a pH of about 6.8.

11. The composition for use according to any one of the preceding claims, wherein the liposomal composition comprises at least one of a pharmaceutically acceptable additive, a carrier, an excipient and a diluent.

12. The composition for use according to any one of the preceding claims, wherein the method of treating Parkinson's disease (PD) is selected from reducing tremor, increasing physical movement, increasing walking speed, improving walking ability, improving motion control, improvement of speech changes, in a patient with Parkinson's disease (PD).

13. A liposomal composition for use in a method of treating Parkinson's disease (PD), said liposomal composition comprising sphingomyelin in a lipid bilayer and a therapeutically effective amount of monosialotetrahexosylganglioside (GM1), wherein the monosialotetrahexosylganglioside (GM1) is partially located in a membrane and partially located in an aqueous core of liposomes, wherein the treatment optionally involves a dosing regimen with a therapeutically effective dose of said liposomal composition being administered in a primary mode of administration at most every 4 days with at least 3 days between each administration; preferably at most every 6 days with at least 5 days between each administration; most preferably at most every 7 days with at least 6 days between each administration; **characterised in that** the treatment is an at least partial reversal of Parkinson's disease (PD); wherein the composition is administered intravenously in the primary mode of administration.

## Patentansprüche

1. Liposomale Zusammensetzung zur Verwendung in einem Verfahren zur Behandlung der Parkinson-Krankheit (PD), wobei die liposomale Zusammensetzung Sphingomyelin in einer Lipiddoppelschicht und eine therapeutisch wirksame Menge an Monosialotetrahexosylgangliosid (GM1) umfasst, wobei das Monosialotetrahexosylgangliosid (GM1) teilweise in einer Membran und teilweise in einem wässrigen Kern von Liposomen lokalisiert ist,
**dadurch gekennzeichnet, dass** eine therapeutisch wirksame Dosis der liposomalen Zusammensetzung höchstens alle 4 Tage in einem primären Verabreichungsmodus mit mindestens 3 Tagen zwischen jeder Verabreichung verabreicht wird; vorzugsweise höchstens alle 6 Tage in einem primären Verabreichungsmodus mit mindestens 5 Tagen zwischen jeder Verabreichung; am meisten bevorzugt höchstens alle 7 Tage in einem primären Verabreichungsmodus mit mindestens 6 Tagen zwischen jeder Verabreichung;
wobei die Zusammensetzung im primären Verabreichungsmodus intravenös verabreicht wird.

2. Zusammensetzung zur Verwendung nach Anspruch 1, wobei die Dosis über einen Zeitraum von zwischen 45 Minuten und 90 Minuten, vorzugsweise etwa 1 Stunde, verabreicht wird.

3. Zusammensetzung zur Verwendung nach einem der vorhergehenden Ansprüche, wobei die Verabreichung im primären Verabreichungsmodus von einer Verabreichung in einem sekundären Verabreichungsmodus zwischen den Dosen des primären Verabreichungsmodus begleitet wird, wobei der sekundäre Verabreichungsmodus vorzugsweise eine orale Verabreichung ist.

4. Zusammensetzung zur Verwendung nach einem der vorhergehenden Ansprüche, wobei die Verabreichung des primären und/oder sekundären Verabreichungsmodus ein Dosierungsschema mit gleichen Dosen oder steigenden Dosen der liposomalen Zusammensetzung, die GM1 umfasst, umfasst.

5. Zusammensetzung zur Verwendung nach einem der Ansprüche 1 bis 3, wobei der primäre Verabreichungsmodus eine zweite Dosis der liposomalen Zusammensetzung, die GM1 umfasst, die niedriger als die erste Dosis ist, und/oder die zweite Dosis mit einer niedrigeren Flussrate als die erste Dosis verabreicht wird, umfasst.

6. Zusammensetzung zur Verwendung nach einem der vorhergehenden Ansprüche, wobei die Zusammensetzung periodisch alle 7 Tage mit 6 Tagen zwischen jeder Verabreichung im primären Verabreichungsmodus verabreicht wird.

7. Zusammensetzung zur Verwendung nach einem der vorhergehenden Ansprüche, wobei die therapeutisch wirksame Dosis von GM1 der liposomalen Zusammensetzung zwischen 300 mg und 800 mg, vorzugsweise zwischen 600 mg und 750 mg liegt und am meisten bevorzugt etwa 720 mg ist.

8. Zusammensetzung zur Verwendung nach einem der vorhergehenden Ansprüche, wobei die liposomale Zusammensetzung zusätzlich Cholesterin, vorzugsweise Sphingomyelin und Cholesterin in einem molaren Verhältnis von 1:1, umfasst.

9. Zusammensetzung zur Verwendung nach einem der vorhergehenden Ansprüche, wobei die Liposomen der liposomalen Zusammensetzung
- einen mittleren Durchmesser zwischen 10 nm und 70 nm, vorzugsweise zwischen 30 nm und 70 nm, stärker bevorzugt zwischen 40 nm und 65 nm, gemessen mittels dynamischer Lichtstreuung, aufweisen; und/oder
- einen mittleren Durchmesser zwischen 10 nm und 50 nm, vorzugsweise zwischen 20 nm und 50 nm, stärker bevorzugt zwischen 30 nm und 40 nm, gemessen mittels Kryo-TEM, aufweisen.

10. Zusammensetzung zur Verwendung nach einem der vorhergehenden Ansprüche, wobei die liposomale Zusammensetzung phosphatgepufferte Salzlösung bei einem pH-Wert von etwa 6,8 umfasst.

11. Zusammensetzung zur Verwendung nach einem der vorhergehenden Ansprüche, wobei die liposomale Zusammensetzung mindestens einen pharmazeutisch annehmbaren Zusatzstoff, einen Träger, einen Hilfsstoff und/oder ein Verdünnungsmittel umfasst.

12. Zusammensetzung zur Verwendung nach einem der vorhergehenden Ansprüche, wobei das Verfahren zur Behandlung der Parkinson-Krankheit (PD) ausgewählt ist aus der Reduzierung von Tremor, der Erhöhung der körperlichen Bewegung, der Erhöhung der Gehgeschwindigkeit, der Verbesserung der Gehfähigkeit, der Verbesserung der Bewegungskontrolle, der Verbesserung von Sprachveränderungen bei einem Patienten mit Parkinson-Krankheit (PD).

13. Liposomale Zusammensetzung zur Verwendung in einem Verfahren zur Behandlung der Parkinson-Krankheit (PD), wobei die liposomale Zusammensetzung Sphingomyelin in einer Lipiddoppelschicht und eine therapeutisch wirksame Menge an Monosialotetrahexosylgangliosid (GM1) umfasst, wobei das Monosialotetrahexosylgangliosid (GM1) teilweise in einer Membran und teilweise in einem wässrigen Kern von Liposomen lokalisiert ist, wobei die Behandlung optional ein Dosierungsschema umfasst, bei dem eine therapeutisch wirksame Dosis der liposomalen Zusammensetzung in einem primären Verabreichungsmodus höchstens alle 4 Tage mit mindestens 3 Tagen zwischen jeder Verabreichung verabreicht wird; vorzugsweise höchstens alle 6 Tage mit mindestens 5 Tagen zwischen jeder Verabreichung; am meisten bevorzugt höchstens alle 7 Tage mit mindestens 6 Tagen zwischen jeder Verabreichung; **dadurch gekennzeichnet, dass** die Behandlung eine zumindest teilweise Umkehrung der Parkinson-Krankheit (PD) ist; wobei die Zusammensetzung im primären Verabreichungsmodus intravenös verabreicht wird.

## Revendications

1. Une composition liposomale destinée à être utilisée dans une méthode de traitement de la maladie de Parkinson (MP), ladite composition liposomale comprenant de la sphingomyéline dans une bicouche lipidique et une quantité thérapeutiquement efficace de monosialotétrahexosylganglioside (GM1), dans laquelle le monosialotétrahexosylganglioside (GM1) est partiellement situé dans une membrane et partiellement situé dans un noyau aqueux de liposomes, **caractérisée en ce qu'**une dose thérapeutiquement efficace de ladite composition liposomale est administrée au plus tous les 4 jours selon un mode d'administration primaire avec au moins 3 jours entre chaque administration; de préférence au plus tous les 6 jours selon un mode d'administration primaire avec au moins 5 jours entre chaque administration; plus préférablement au plus tous les 7 jours selon un mode d'administration primaire avec au moins 6 jours entre chaque administration;
dans laquelle la composition est administrée par voie intraveineuse selon le mode d'administration primaire.

2. La composition destinée à être utilisée selon la revendication 1, dans laquelle la dose est administrée sur une période comprise entre 45 minutes et 90 minutes, de préférence environ 1 heure.

3. La composition destinée à être utilisée selon l'une quelconque des revendications précédentes, dans laquelle l'administration selon le mode d'administration primaire est accompagnée d'une administration selon un mode d'administration secondaire entre les doses du mode d'administration primaire, dans laquelle le mode d'administration secondaire est de préférence une administration orale.

4. La composition destinée à être utilisée selon l'une quelconque des revendications précédentes, dans laquelle l'administration du mode d'administration primaire et/ou secondaire comprend un régime posologique de doses égales, ou de doses croissantes de ladite composition liposomale comprenant du GM1.

5. La composition destinée à être utilisée selon l'une quelconque des revendications 1 à 3, dans laquelle le mode d'administration primaire comprend une deuxième dose de ladite composition liposomale comprenant du GM1 qui est inférieure à la première dose et/ou la deuxième dose est administrée à un débit plus faible que la première dose.

6. La composition destinée à être utilisée selon l'une quelconque des revendications précédentes, dans laquelle ladite composition est administrée périodiquement tous les 7 jours avec 6 jours entre chaque administration selon le mode d'administration primaire.

7. La composition destinée à être utilisée selon l'une quelconque des revendications précédentes, dans laquelle ladite dose thérapeutiquement efficace de GM1 de ladite composition liposomale est comprise entre 300 mg et 800 mg, de préférence entre 600 mg et 750 mg, plus préférablement environ 720 mg.

8. La composition destinée à être utilisée selon l'une quelconque des revendications précédentes, dans laquelle la composition liposomale comprend en outre du cholestérol, de préférence de la sphingomyéline et du cholestérol dans un rapport molaire de 1:1.

9. La composition destinée à être utilisée selon l'une quelconque des revendications précédentes, dans laquelle les liposomes de ladite composition liposomale
- ont un diamètre moyen compris entre 10 nm et 70 nm, de préférence entre 30 nm et 70 nm, plus préférablement entre 40 nm et 65 nm, mesuré par diffusion dynamique de la lumière; et/ou
- ont un diamètre moyen compris entre 10 nm et 50 nm, de préférence entre 20 nm et 50 nm, plus préférablement entre 30 nm et 40 nm, mesuré par CryoTEM.

10. La composition destinée à être utilisée selon l'une quelconque des revendications précédentes, dans laquelle la composition liposomale comprend une solution saline tamponnée au phosphate à un pH d'environ 6,8.

11. La composition destinée à être utilisée selon l'une quelconque des revendications précédentes, dans laquelle la composition liposomale comprend au moins l'un parmi un additif pharmaceutiquement acceptable, un véhicule, un excipient et un diluant.

12. La composition destinée à être utilisée selon l'une quelconque des revendications précédentes, dans laquelle la méthode de traitement de la maladie de Parkinson (MP) est choisie parmi la réduction des tremblements, l'augmentation des mouvements physiques, l'augmentation de la vitesse de marche, l'amélioration de la capacité de marche, l'amélioration du contrôle des mouvements, l'amélioration des changements de la parole, chez un patient atteint de la maladie de Parkinson (MP).

13. Une composition liposomale destinée à être utilisée dans une méthode de traitement de la maladie de Parkinson (MP), ladite composition liposomale comprenant de la sphingomyéline dans une bicouche lipidique et une quantité thérapeutiquement efficace de monosialotétrahexosylganglioside (GM1), dans laquelle le monosialotétrahexosylganglioside (GM1) est partiellement situé dans une membrane et partiellement situé dans un noyau aqueux de liposomes, dans laquelle le traitement implique facultativement un régime posologique avec une dose thérapeutiquement efficace de ladite composition liposomale étant administrée selon un mode d'administration primaire au plus tous les 4 jours avec au moins 3 jours entre chaque administration; de préférence au plus tous les 6 jours avec au moins 5 jours entre chaque administration; plus préférablement au plus tous les 7 jours avec au moins 6 jours entre chaque administration; **caractérisée en ce que** le traitement est une inversion au moins partielle de la maladie de Parkinson (MP);
dans laquelle la composition est administrée par voie intraveineuse selon le mode d'administration primaire.
